# EUROPÄISCHE PATENTSCHRIFT

(11) **EP 0 392 214 B1**
(45) Veröffentlichungstag und Bekanntmachung des Hinweises auf die Patenterteilung: **08.12.1993**
(21) Anmeldenummer: 90105048.4
(22) Anmeldetag: 17.03.1990
(51) Int. Cl.: A61M 5/168

(54) **Tropfkammer für Übertragungsgeräte**
Drip chamber for transfer devices
Chambre de gouttes pour appareils de transfert

(30) Priorität: 12.04.1989 DE 3911968
(43) Veröffentlichungstag der Anmeldung: 17.10.1990
(73) Patentinhaber: CLINICO INFUSIONSTECHNIK GmbH, D-36222 Bad Hersfeld (DE)
(72) Erfinder: Heinzerling, Walter C., D-6430 Bad Hersfeld (DE); Heinzerling, Jörg, Dip.-Ing., D-6430 Bad Hersfeld (DE)
(74) Vertreter: von Raffay, Vincenz, Dipl.-Ing.

(56) Entgegenhaltungen:
- FR-A- 1 075 682
- US-A- 4 037 597
- US-A- 4 055 176

## Beschreibung

Die Erfindung betrifft eine Tropfkammer für Übertragungsgeräte nach dem Oberbegriff des Anspruchs 1.

Eine derartige Tropfkammer ist aus der Lehre der EP-A-7 601 vorbekannt. Gemäß der dortigen Beschreibung und den Ansprüchen wird es als zwingend erforderlich angesehen, das Tropfkammerunterteil aus einem Gemisch oder einer Legierung von
a) Polystyrol- Homo- oder Copolymerisaten mit
b) thermoplastischen Kautschuken auf der Basis von Styrol-Butadien-Styrol oder Styrol-Isopren-Styrol-Blockpolymerisaten
herzustellen, um die gewünschte Transparenz, Flexibilität und Beständigkeit gegen energiereiche Strahlung, sowie integrierte Anordnung von Tropfkammerunterteil und Schlauchansatz und im Hinblick auf die Verschweißbarkeit miteinander verträglichen Kunststoffmaterialien für Tropfkammeroberteil und - letztendlich - die integrierte Anordnung von Tropfkammeroberteil und Einstechteil zu erreichen.

Bei der Herstellung des Tropfkammer-Unterteils, das üblicherweise im Spritzgusverfahren gefertigt wird, kommt es je nach Wahl des Angusses zu mehr oder weniger stark ausgeprägten Bereichen, in denen das während des Einspritzvorganges noch schmelzflüssige Material von mehreren Seiten her kommend zusammenfließt. Diese als Fließnähte bezeichneten Bereiche stellen besondere Schwachpunkte dar, insbesondere, wenn bedingt durch eine Materialinhomoginität ein vollständiges Verschweißen der Fließfronten nicht gewährleistet ist. Solche Inhomogenitäten treten bei mechanischen Mischvorgängen auf, wenn die Einzelkunststoffe nicht ausreichend miteinander vermischt werden, oder die Mischungsverhältnisse fehlerhaft sind. Die oben genannten Fließnähte können bei mechanischer Belastung des Bauteils aufbrechen und damit den Bediener (Infektion, Verätzung) oder den Patienten (Luftembolie) gefährden.

Weiter ist ein glasklares und zugleich hochflexibles Tropfkammer-Unterteil zum leichten und schnellen Einstellen sowie Ablesen der gewünschten Tropfenrate erforderlich. Elektronische Tropfenzähl- und Regelgeräte sowie Infusionspumpen arbeiten zur Erfassung der Tropfenrate mit Lichtschranken, die auf eine maximale Transparenz, wie sie nur ein glasklares Bauteil bieten kann, angewiesen sind. Ein Kunststoffgemisch, wie aus der Lehre der EP-A-7601 bekannt, kann nicht glasklar sein und bewirkt dadurch eine stark erhöhte Fehlerwahrscheinlichkeit der oben geannten Geräte.

Ebenso war die vorbekannte Tropfkammer darin nachteilig, daß ihr Unterteil hinsichtlich seiner Flexibilität verbesserungswürdig erschien.

Der vorliegenden Erfindung liegt deshalb die Aufgabe zugrunde, die eingangs genannte Tropfkammer derart zu verbessern, daß sie die geschilderten Nachteile nicht aufweist und eine glasklare Durchsicht durch das Unterteil zu ermöglichen, um eine ausgezeichnete Patientensicherheit, z.B. für die Erkennung von Luftblasen bzw. der richtigen Einstellung der Tropfenrate, zu gewährleisten und gleichzeitig die Flexibilität des Unterteils zu erhöhen.

Diese Aufgabe wird durch die im Anspruch 1 gekennzeichnete Tropfkammer gelöst. Obwohl der Stand der Technik also ein Polymerisatgemisch aus den erwähnten Stoffgruppen a) und b) als unbedingt erforderlich ansah, schafft die Erfindung hier Abhilfe, indem sie das Unterteil ausschließlich aus Styrol-Butadien-Copolymerisat vorsieht, so daß jegliche Mischvorgänge überflüssig werden. Gleichzeitig wird die Wandstärke des Unterteils reduziert, wodurch seine Flexibilität bis hin zur glasklaren Durchsichtigkeit verbessert wird. Überraschenderweise führt die Kombination dieser beiden Merkmale zu einer Tropfkammer mit ausgezeichneten Eigenschaften, obwohl die Lehre der eingangs genannten EP-A-7 601 gerade den Einsatz von Polymergemischen für unabdingbar hält und insbesondere in Spalte 3, Zeilen 15 ff., Styrol-Butadien-Pfropfpolymerisate als völlig ungeeignet darstellt.

Weitere Vorteile und Merkmale gehen aus den nachstehenden Unteransprüchen hervor, die einzeln oder auch gemeinsam mit dem Gegenstand des Hauptanspruchs von erfinderischer Bedeutung sein können.

Im folgenden werden anhand der Zeichnungen fünf verschiedene, bevorzugte Ausführungsbeispiele beschrieben, die zum besseren Verständnis der Erfindung dienen sollen.

Es zeigt:
- Fig. 1: eine Querschnittsansicht eines Unterteils einer erfindungsgemäßen Tropfkammer;
- Fig. 2: eine vergrößerte Darstellung der Fig. 1 im Ausschnitt;
- Fig. 3: eine weitere Ausführungsform eines Unterteils der erfindungsgemäßen Tropfkammer im Querschnitt;
- Fig. 4: einen vergrößerten Ausschnitt des in Fig. 3 gezeigten Unterteils;
- Fig. 5: eine noch weitere Ausführungsform eines Unterteils der erfindungsgemäßen Tropfkammer im Querschnitt;
- Fig. 6: einen vergrößerten Ausschnitt des in Fig. 5 gezeigten Unterteils;
- Fig. 7: eine noch weitere Ausführungsform eines Unterteils der erfindungsgemäßen Tropfkammer im Querschnitt;
- Fig. 8: einen vergrößerten Ausschnitt des in Fig. 7 gezeigten Unterteils;
- Fig. 9: eine noch weitere Ausführungsform eines Unterteils der erfindungsgemäßen Tropfkammer im Querschnitt;
- Fig.10: einen vergrößerten Ausschnitt des in Fig. 9 gezeigten Unterteils.

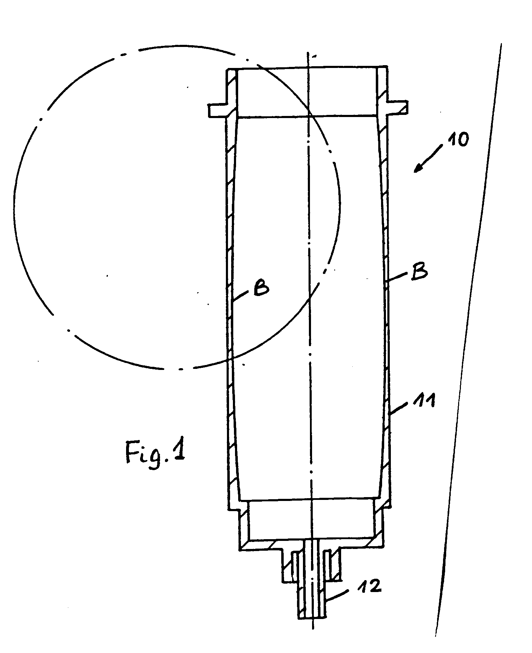

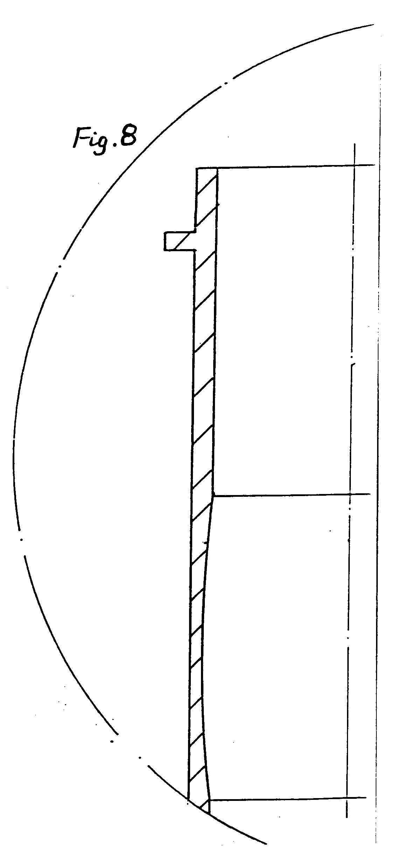

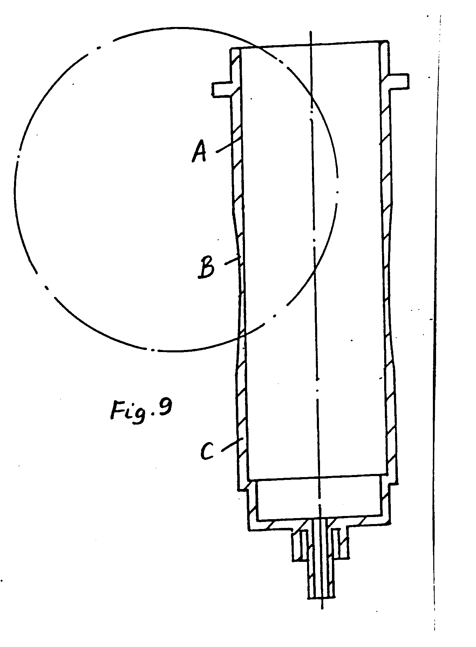

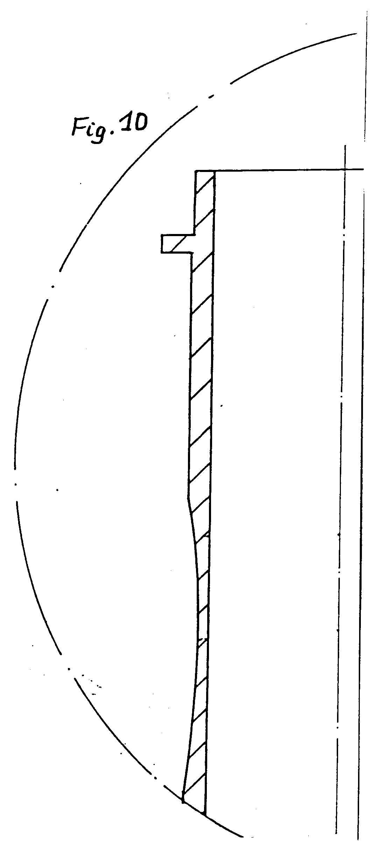

In Fig. 1 ist das Tropfkammerunterteil allgemein mit 10 bezeichnet. Es weist eine zylindrische Form auf, mit einer in etwa kreisförmigen Wandung 11 und einem Schlauchansatzstück 12. Sämtliche in den Fig. 1 bis 10 gezeigten Unterteile 10 bestehen ausschließlich aus einem einheitlichen Styrol-Butadien-Copolymerisat und weisen sämtliche eine in ihrer Querschnittsdicke reduzierte Wandstärke auf.

Deutlich ist aus Fig. 2 zu ersehen, daß die Endbereiche A und C der Wandung 11 im Querschnitt wesentlich dicker sind als der mittlere Bereich B. In der Ausführungsform der Fig. 2 ist die Reduzierung der Wandstärke durch eine konkave Form der Innenwandung erzielt worden.

Die Fig. 3 und 4 zeigen hingegen eine Ausführungsform, bei der eine noch stärkere Reduzierung der Wandstärke im Vergleich zu den Fig. 1 und 2 erzielt wird, und zwar durch eine konkave Form der Außenwandung.

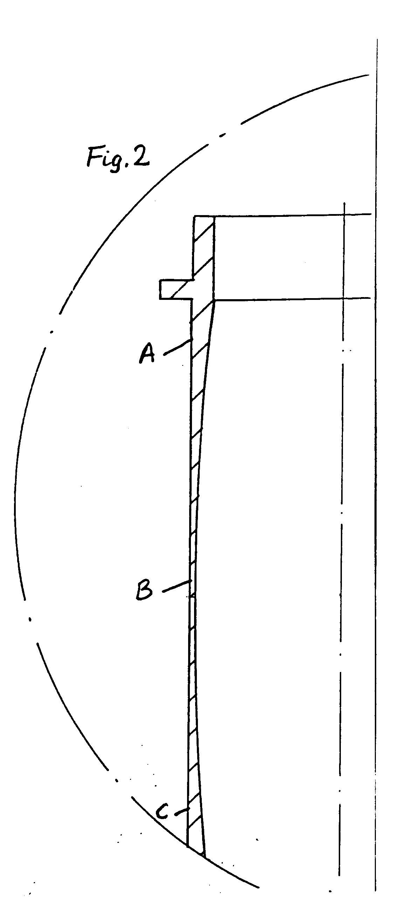

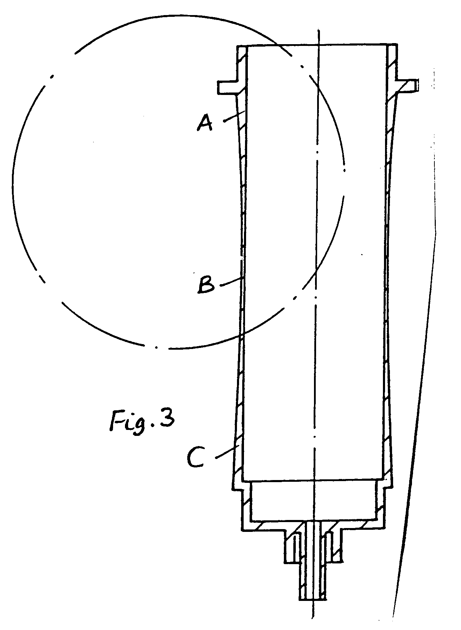

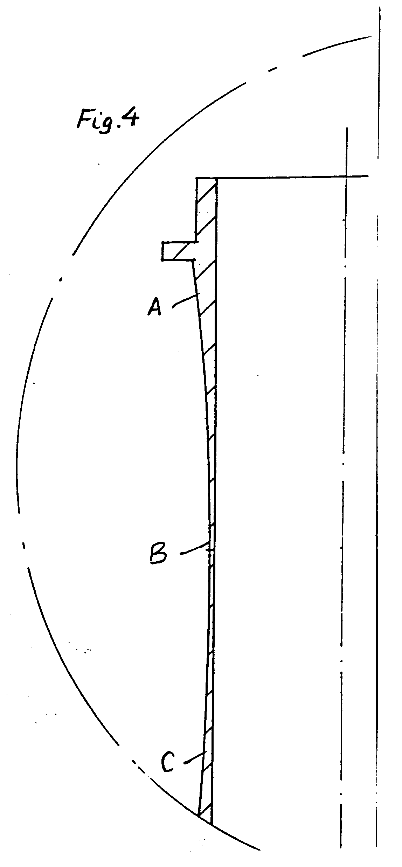

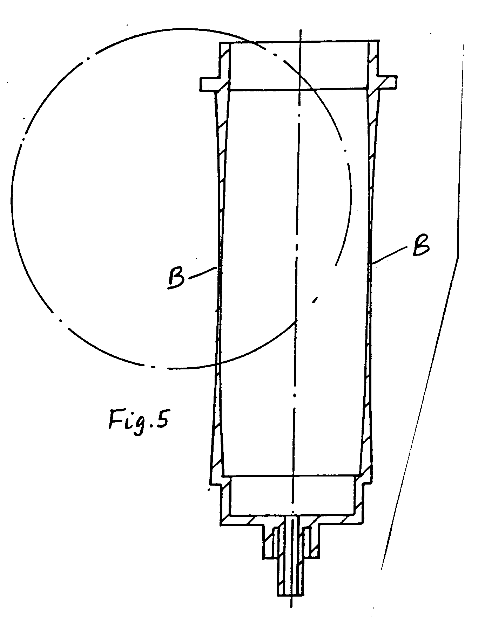

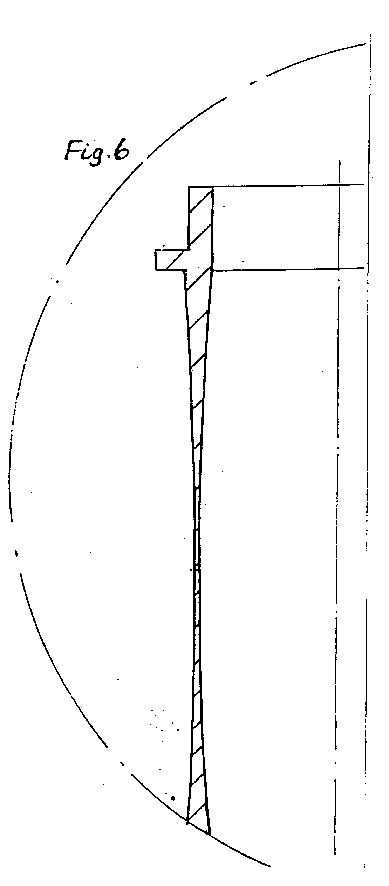

Die Fig. 5 und 6 zeigen eine den Fig. 3 und 4 vergleichbare Ausführungsform mit noch stärkerer Reduzierung der Wandstärke in bikonkaver Form im Bereich B, die nur noch 30 % der mittleren Wandstärke von etwa 0,6 bis 0,75 mm beträgt. Eine weitere Reduzierung sollte aus Festigkeitsgründen nicht vorgenommen werden. Der günstigste Wert der Wandstärke im mittigen Bereich B liegt bei etwa 50 % der mittleren Wandstärke in den Endbereichen A und C.

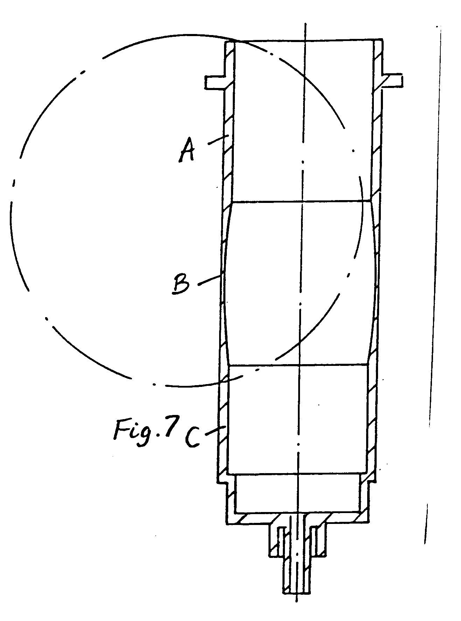

Während in den bisher geschilderten Ausführungsformen die Reduzierung der Wandstärke im wesentlichen kontinuierlich über die Gesamtlänge der Wandung zur Mitte hin zunimmt und im mittigen Bereich B am deutlichsten hervortritt, zeigen die weiteren Ausführungsformen gemäß den Fig. 7 bis 10 zwei Ausführungsformen, in denen die Endbereiche A und C, die jeweils ein Drittel der Gesamtlänge der Seitenwand umfassen, eine unveränderte Struktur, ohne Reduzierung der Wandstärke, die dort ausschließlich im mittleren Bereich B vorgesehen ist.

## Patentansprüche

1. Tropfkammer für Übertragungsgeräte mit einem Oberteil aus Polystyrol-Homo- oder Copolymerisaten und einem damit verbundenen flexiblen glasklaren Unterteil aus Copolymerisat, dadurch gekennzeichnet, daß das Untertell ausschließlich aus Styrol-Butadien-Copolymerisat besteht, und daß das Unterteil in seinem mittigen Bereich (B) eine Wandung mit reduzierter Wandstärke aufweist.

2. Tropfkammer nach Anspruch 1, dadurch gekennzeichnet, daß das zur Herstellung verwendete Styrol-Butadien-Copolymerisat Elastomerteilchen mit einer maximalen Größe von 1 µm aufweist.

3. Tropfkammer nach Anspruch 1, dadurch gekennzeichnet, daß die Wandstärke im mittigen Bereich (B) 30 bis 70% der mittleren Wandstärke von etwa 0,6 bis 0,75 mm in den Endbereichen (A,C) des Unterteils beträgt.

4. Tropfkammer nach Anspruch 3, dadurch gekennzeichnet, daß die Wandstärke im Bereich (B) 45 bis 55 % beträgt.

5. Tropfkammer nach Anspruch 1, dadurch gekennzeichnet, daß das Styrol-Butadien-Copolymerisat ein Pfropfpolymerisat darstellt.

6. Tropfkammer nach Anspruch 1 und 4, dadurch gekennzeichnet, daß das Styrol-Butadien-Copolymerisat mittels Lösungsmittelpolymerisation hergestellt ist.

7. Tropfkammer nach Anspruch 1 bis 6, dadurch gekennzeichnet, daß die Wandung des Unterteils im Querschnitt eine konkave oder bikonkave Form aufweist.

8. Tropfkammer nach Anspruch 1 bis 7, dadurch gekennzeichnet, daß die Wandstärke kontinuierlich über die Gesamtlänge hin zur Mitte des Bereichs (B) abnimmt.

## Claims

1. Drip chamber for transfer equipment with a top part made from polystyrene homo- or copolymers and a copolymer, flexible, glass-clear bottom part connected thereto, characterized in that the bottom part is exclusively made from styrene-butadiene copolymer and that the central region (B) of the bottom part has a wall with a reduced thickness.

2. Drip chamber according to claim 1, characterized in that the styrene-butadiene copolymer used for production purposes contains elastomer particles with a maximum size of 1 µm.

3. Drip chamber according to claim 1, characterized in that in the central region (B) the wall thickness is 30 to 70% of the average wall thickness of approximately 0.6 to 0.75 mm in the end regions (A, C) of the bottom part.

4. Drip chamber according to claim 3, characterized in that the wall thickness in region (B) is 45 to 55%.

5. Drip chamber according to claim 1, characterized in that the styrene-butadiene copolymer is a graft polymer.

6. Drip chamber according to claims 1 and 4, characterized in that the styrene-butadiene copolymer is produced by means of solvent polymerization.

7. Drip chamber according to claims 1 to 6, characterized in that the wall of the bottom part has, in cross-section, a concave or biconcave shape.

8. Drip chamber according to claims 1 to 7, characterized in that the wall thickness continuously decreases over the total length up to the centre of the region (B).

## Revendications

1. Chambre de gouttes pour appareils de transfert comprenant une partie supérieure constituée d'homopolymères ou de copolymères de polystyrène et une partie inférieure claire et flexible reliée à la partie supérieure et constituée d'un copolymère, **caractérisée** par le fait que la partie inférieure est constituée exclusivement d'un copolymère de styrène-butadiène et que la partie inférieure présente dans sa région centrale (B) une paroi à épaisseur réduite.

2. Chambre de gouttes selon la revendication 1, **caractérisée** par le fait que le copolymère de styrène-butadiène utilisé pour la fabrication présente des particules d'élastomère d'une taille maximale de 1 µm.

3. Chambre de gouttes selon la revendication 1, **caractérisée** par le fait que l'épaisseur de paroi dans la région centrale (B) représente 30 à 70% de l'épaisseur de paroi moyenne comprise entre environ 0,6 et 0,75 mm dans les régions terminales (A, C) de la partie inférieure.

4. Chambre de gouttes selon la revendication 3, **caractérisée** par le fait que l'épaisseur de paroi dans la région (B) représente entre 45 et 55%.

5. Chambre de gouttes selon la revendication 1, **caractérisée** par le fait que le copolymère de styrène-butadiène est un copolymère implanté.

6. Chambre de gouttes selon les revendications 1 et 4, **caractérisée** par le fait que le copolymère de styrène-butadiène est fabriqué par polymérisation par solvant.

7. Chambre de gouttes selon les revendications 1 à 6, **caractérisée** par le fait que la paroi de la partie inférieure présente une section transversale concave ou biconcave.

8. Chambre de gouttes selon la revendication 1 à 7, **caractérisée** par le fait que l'épaisseur de paroi diminue en continu sur toute la longueur vers le milieu de la région (B).
